# EUROPEAN PATENT SPECIFICATION

(11) **EP 1 597 393 B1**
(45) Date of publication and mention of the grant of the patent: **30.11.2011**
(21) Application number: 04713289.9
(22) Date of filing: 20.02.2004
(51) Int. Cl.: C12Q 1/68

(54) **METHODS OF DETERMINING A CHEMOTHERAPEUTIC REGIMEN BASED ON LOSS OF HETEROZYGOSITY AT THE THYMIDYLATE SYNTHASE LOCUS**
VERFAHREN ZUR PROGNOSTISCHEN BESTIMMUNG VON CHEMOTHERAPIE, BASIEREND AUF EINEM VERLUST AN HETEROZYGOSITÄT (LOH) DES THYMIDILATSYNTHASE LOCUS
METHODE D'ELABORATION D'UN TRAITEMENT DE CHIMIOTHERAPIE EN FONCTION D'UNE BAISSE DE L'HETEROZYGOSITE DETECTEE AU NIVEAU DES LOCUS DE LA THYMIDYLATE SYNTHASE

(30) Priority: 27.02.2003 US 373752
(43) Date of publication of application: 23.11.2005
(73) Proprietor: Response Genetics, Inc., Los Angeles, CA 90033 (US)
(72) Inventor: DANENBERG, Kathleen, D., Altadena, CA 91001 (US)
(74) Representative: Furlong, Isla Jane
(86) International application number: PCT/US2004/004867
(87) International publication number: WO 2004/075833

(56) References cited:
- KAWAKAMI K ET AL.: "Functional polymorphism of the thymidylate synthase gene in colorectal cancer accompanied by frequent loss of heterozygosity" JAPANESE JOURNAL OF CANCER, vol. 93, November 2002 (2002-11), pages 1221-1229, XP008033672
- KOHYA N ET AL.: "Mutation analysis of K-ras and beta-cateningenes related to O6-methylguanin-DNA methyltransferase and mismatch repair protein status in human gallbladder carcinoma" INTERNATIONAL JOURNAL OF MOLECULAR MEDICINE, vol. 11, January 2003 (2003-01), pages 65-69, XP002294281
- SHIROTA Y ET AL: "ERCC1 AND THYMIDYLATE SYNTHASE MRNA LEVELS PREDICT SURVIVAL FOR COLORECTAL CANCER PATIENTS RECEIVING COMBINATION OXALIPLATIN AND FLUOROURACIL CHEMOTHERAPY" JOURNAL OF CLINICAL ONCOLOGY, GRUNE AND STRATTON, NEW YORK, NY, US, vol. 19, no. 23, 1 December 2001 (2001-12-01), pages 4298-4304, XP009002481 ISSN: 0732-183X
- BASILION J P ET AL: "SELECTIVE KILLING OF CANCER CELLS BASED ON LOSS OF HETEROZYGOSITY AND NORMAL VARIATION IN THE HUMAN GENOME: A NEW PARADIGM FOR ANTICANCER DRUG THERAPY" MOLECULAR PHARMACOLOGY, BALTIMORE, MD, US, vol. 56, no. 2, August 1999 (1999-08), pages 359-369, XP001117596 ISSN: 0026-895X
- KAWAKAMI K ET AL.: "Functional polymorphism of the thymidylate synthase gene in colorectal cancer is modulated by frequent loss of heterozygosity" PROCEEDINGS OF THE AMERICAN ASSOCIATION FOR CANCER RESEACH, vol. 43, March 2002 (2002-03), page 320, XP008033671
- IACOPETTA B ET AL.: "A polymorphism in the enhancer region of the thymidylate synthase promoter influences the survival of colorectal cancer patients treated with 5-fluorouracil" BRITISH JOURNAL OF CANCER, vol. 85, no. 6, 2001, pages 827-830, XP001199503 cited in the application
- UCHIDA K ET AL.: "Loss of heterozygosity at the thymidylate synthase (TS) locus on chromosome 18 affects tumor response and survival in individuals heterozygous for a 28-bp polymorphism in the TS gene" CLINICAL CANCER RESEARCH, vol. 10, 15 January 2004 (2004-01-15), pages 433-439, XP002294282

### SEQUENCE LISTING

<110> DANENBERG, KATHLEEN
<120> METHODS OF DETERMINING A CHEMOTHERAPEUTIC REGIMEN BASED ON LOSS OF HETEROZYGOSITY AT THE THYMIDYLATE SYNTHASE LOCUS
<130> 11220/230
<140>
   <141> 2004-02-20
<160> 6
<170> PatentIn Ver. 2.1
<210> 1
   <211> 18
   <212> DNA
   <213> Artificial Sequence
<220>
   <223> Description of Artificial sequence: Primer
<400> 1
   gcctcggtgt gcctttca 18
<210> 2
   <211> 17
   <212> DNA
   <213> Artificial Sequence
<220>
   <223> Description of Artificial Sequence: Primer
<400> 2
   cccgtgatgt gcgcaat 17
<210> 3
   <211> 21
   <212> DNA
   <213> Artificial Sequence
<220>
   <223> Description of Artificial Sequence: Probe
<400> 3
   tcgccagcta cgccctgctc a 21
<210> 4
   <211> 18
   <212> DNA
   <213> Artificial Sequence
<220>
   <223> Description of Artificial Sequence: Primer
<400> 4 tgagcgcggc tacagctt 18
<210> 5
   <211> 22
   <212> DNA
   <213> Artificial Sequence
<220>
   <223> Description of Artificial Sequence: Primer
<400> 5 tccttaatgt cacgcacgat tt 22
<210> 6
   <211> 18
   <212> DNA
   <213> Artificial Sequence
<220>
   <223> Description of Artificial Sequence: Probe
<400> 6 accaccacgg ccgagcgg 18

## Description

### BACKGROUND OF THE INVENTION

Thymdiylate synthase (TS) catalyzes the reductive methylation of 2'-deoxyuridylate 5,10-methylenetetrahydrofolate to form 2'-thymidylate and dihydrofolate. This is an essential step in DNA biosynthesis. Since TS is the only *de novo* source of the thymine base and its reaction is one of the rate-limiting steps in DNA synthesis, inhibition of TS has been a fruitful approach in cancer chemotherapy (Danenberg, P.V., Biochim. Biophys. Acta, 473: 73-92, 1977). TS is the target enzyme for 5-fluorouracil (5-FU), which for almost 50 years have been one of the mainstay drugs for treatment of many cancers. 5-FU exerts its cytotoxic effect through TS inhibition by forming a stable ternary complex among 5,10-methylenetetrahydrofolate, TS and 5-fluoro-2'deoxyuridylate, the active metabolite of 5-FU. Since the appearance of 5-FU, other fluoropyrimidine-base therapies such as FUdR, UFT, S-1 and capecitabine as well as folate-based TS inhibitors such as ratitrexed, pemetrexed and nolatrexed have been developed. *In vitro* studies have shown that cells become resistant to TS inhibitors by up-regulating TS expression and raising intracellular TS levels (Wang, W., et al., Cancer Res., 61: 55055510, 2001), leading to the expectation that the amount of TS in tumors might be a predictor of response to TS-targeted therapy. Indeed, recent studies have shown that the TS expression does vary considerably among tumors and that sensitivity of various tumors to 5-FU-based chemotherapy is correlated with the intratumoral level of TS (Huang, C.L., et al., Int. J. Oncol.,17: 47-54, 2000; Nishimura, R., et al., Anticancer Res., 19: 5621-5626, 1999; Salonga, D., et al., Clin. Cancer Res., 6: 1322-1327, 2000; Shirota, Y., et al., J. Clin. Oncol., 19: 4298-4304, 2001; Yeh, K.H., et al., Cancer, 82: 1626-1631, 1998.) Moreover, high TS levels in tumors have also been shown to be associated with worse prognosis (Kralovanszky, J., et al., Oncology, 62: 167174, 2002; Nakagawa, T., et al., Lung Cancer, 35: 165-70,2002).

The mechanisms by which TS expression is regulated *in vivo* have not yet been clearly defined. However, it has been suggested that polymorphisms occurring in the TS promoter might constitute one regulatory factor. The TS gene is known to have a unique 28 base pair tandemly repeated sequence in the 5'-untranslated region (5'-UTR) and is polymorphic in the numbers of this repeat (Horie, N., et al., Cell Struct. Funct., 20:191-197,1995). Most individuals have either a double tandem repeat (2R/2R), three repeat (3R/3R) or a heterozygous (2R/3R) genotype, although higher order repeats are found in a few cases (Marsh, S., et al., Genomics, 58: 310-312, 1999). The TS enhancer region (TSER) polymorphism is a partial determinant of TS protein expression in human gastrointestinal cancers (Kawakami, K., et al., Anticancer Res., 19: 3249-3252, 1999), which reference is herein incorporated by reference in its entirety.

Cancer tissue with 3R/3R genotype has showed significantly higher TS protein expression than that with 2R/3R genotype, which has been confirmed with *in vitro* experiments (Kawakami, K., et al., Clin. Cancer Res., 7: 4096-4101, 2001.), which reference is herein incorporated by reference in its entirety. This association between TS genotype and TS expression, together with the role of TS expression in a 5-FU-based chemotherapy, suggests that the TS genotype with respect to the number of tandem repeats might be at least a partial predictor for 5-FU-based chemotherapy. Indeed, several recent clinical correlative studies have obtained preliminary evidence that the TS genotype of this TSER is associated with response and survival of colorectal cancer patients treated with 5-FU-based therapies (Iacopetta, B., et al., Br. J. Cancer, 85: 827-830, 2001; Marsh, S., et al., Int. J. Oncol., 19: 383-386, 2001; Pullarkat, S. et al., Pharmacogenomics J., 1, 65-70, 2001; Villafranca, E., et al., J. Clin. Oncol., 19: 1779-1786, 2001).

The presence of variable numbers of a 28 base pair tandem repeat sequence in the TSER has drawn considerable interest recently because this is the only genomic lesion besides p53 mutation that may predict to some extent the clinical outcome of patients treated with 5-FU based therapy. All studies done to date agree that possession of the 2R/2R genotype has consistently been associated with greater clinical benefit than the 3R/3R genotype in 5-FU-treated patients (Iacopetta, B., et al., Br. J. Cancer, 85: 827-830, 2001; Pullarkat, S. T. et al., Pharmacogenomics J., 1, 65-70, 2001; Villafranca, E., et al., J. Clin. Oncol., 19: 1779-1786, 2001; Etienne MC, et al., J. Clin. Oncol. 20:2832-43, 2002). The discovery of *TS* polymorphisms that might be tumor response determinants was of considerable interest because of the possibility that prediction of tumor response could be done by analysis of readily available normal tissue (e.g, peripheral blood cells). This expectation is based on the assumption that the genotype in the normal tissue would be identical to that in cancer tissue. However, recently obtained evidence shows that this assumption is not always true in the case of *TS* genotype. A high incidence of LOH has been observed at the TS locus in cancer tissues, which leads to modification of TS genotype in the tumor when it is heterozygous in normal tissue (Kawakami K, et al., Jpn J CancerRes. 93: 1221-1128, 2002 *;* Zinzindohoue F, el al., J. Clin. Oncol. 19 : 3442,2001). That is, the occurrence of LOH in individuals who have a heterozygous 2R/3R genotype in their normal tissue would give rise to a tumor with either a 2R/loss or the 3R/loss TSER genotype. Thus, it is possible that patients who are heterozygous and who have LOH at the TS locus in their tumor tissue might experience considerably different outcomes from chemotherapy depending on which allele became deleted during the LOH event.

Basilion et al, Molecular Pharmacology, vol. 56, no.2, 1999, pp 359-369 describes the selective killing of cancer cells based on LOH occurring during oncogenesis.

Thus, there remains a need for a method to predict a response to various chemotherapuetic regimens prior to treatment. An accurate prediction will allow a physician to determine whether to go forward with a desired chemotherapeutic regimen or to try an alternative chemotherapeutic regimen. Since adverse side affects are prevalent with most chemotherapeutic regimens, it is desirable to be able to predict tumor response to the chemotherapeutic agent so as to eliminate any unnecessary or unsuccessful treatments. Further, if a physician is able to predict the response to a treatment, time will not be wasted on those treatments that are likely to fail and instead allow the physician to focus on more promising treatments. A method of predicting a response to treatment provides a physician with guidelines in choosing the therapy rather than a simple trial and error approach.

### SUMMARY OF THE INVENTION

The present invention is directed to a method of predicting a response to a chemotherapeutic regimen based on loss of heterozygosity at the thymidylate synthase locus in cancer tissue. This method comprises determining normal tissue genotype for thymidylate synthase of a patient; determining tumor tissue genotype for thymidylate synthase of said patient; comparing the normal tissue genotype with the tumor tissue genotype; determining whether a loss of heterozygosity at the thymidylate synthase locus has occurred in the tumor tissue based on the comparison of the genotypes; and predicting a response to the chemotherapeutic regimen based on the loss of heterozygosity in the tumor sample.

The present invention also contemplates utilizing the genotype of the normal tissue to help predict the patient's response (as in drug toxicity response) to the chemotherapeutic regimen.

To assist in predicting the responses, the present invention contemplates compiling patient data, such as a database, that references patient data such as, but not limited to, both normal tissue and cancer tissue genotypes, the cancer being treated, the chemotherapeutic regimen administered, the patient's response, i.e. tumor size reduction, if any, tumor growth, increase in metastases, patient survival, chemotherapeutic dose, toxicity data, life span of patient, etc. The data base will assist in predicting the response of a patient by allowing one to compare the patient's factors with previous patient's having like factors (such as normal tissue and cancer tissue genotype, chemotherapeutic regimens, and outcome of treatment).

In both the predicting a response as well as creating the database, fresh or preserved tissue may be used to determine the tissue genotype.

### BRIEF DESCRIPTION OF THE DRAWINGS

Figure 1A shows the structure ofTS 5' flanking region with a 3-repeat sequence. The translated region is indicated by the solid bar. The open bar with arrows in it indicates the TSER. The arrows represent tandemly repeated sequences and a complementary reverse sequence. The numbers indicate nucleotide position when the first nucleotide of the initiation codon is defined to be +1. The PCR primers are designed to flank the region of the tandem repeats, so that the presence of 3 repeats will give a longer PCR product than a 2-repeat TSER.

Figure 1B shows examples of TS genotype analysis in matched normal (N) and tumor (T) DNA from colorectal cancer patients. The upper and lower bands represent PCR products from amplification of the TSER segment containing 3R and 2R, respectively. The numbers refer to different patient cases. Each patient has a heterozygous 2R/3R genotype in normal tissue, as indicated by the presence of both bands. Case 1: LOH gives rise to a tumor with a 3R/loss genotype. Case 2: LOH does not happen. Case 3: LOH gives rise to a tumor with a loss/2R genotype.

Figure 2 depicts survival (Kaplan-Meier) plots indicating probability of survival for patients with each of the various possible tumor TSER genotypes separately.

Figure 3 depicts survival (Kaplan-Meier) plots indicating probability of survival for patients grouped according to 2R genotype only (2R/loss + 2R/3R), 3R genotype only (3R/3R + 3R/loss) and heterozygotic (2R/3R).

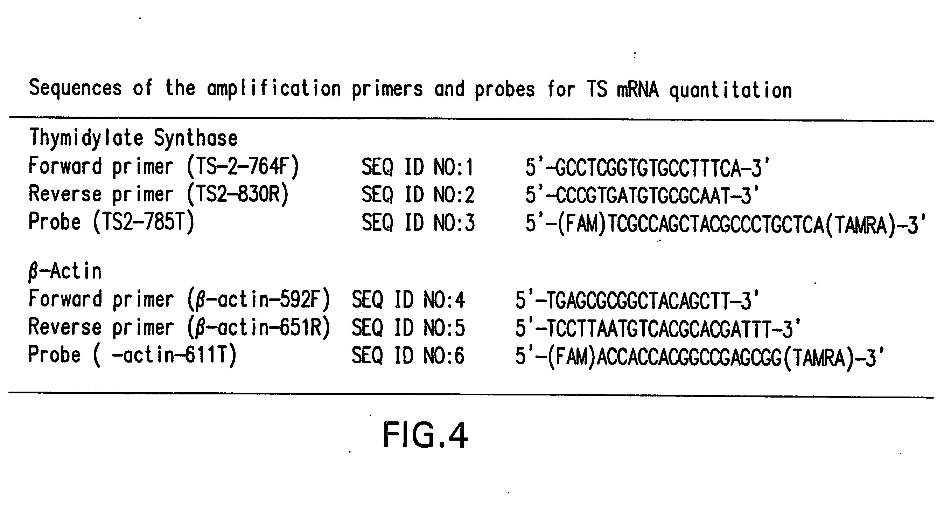

Figure 4 shows the sequences of the amplification primers and probes for TS mRNA quantitation (Table 1).

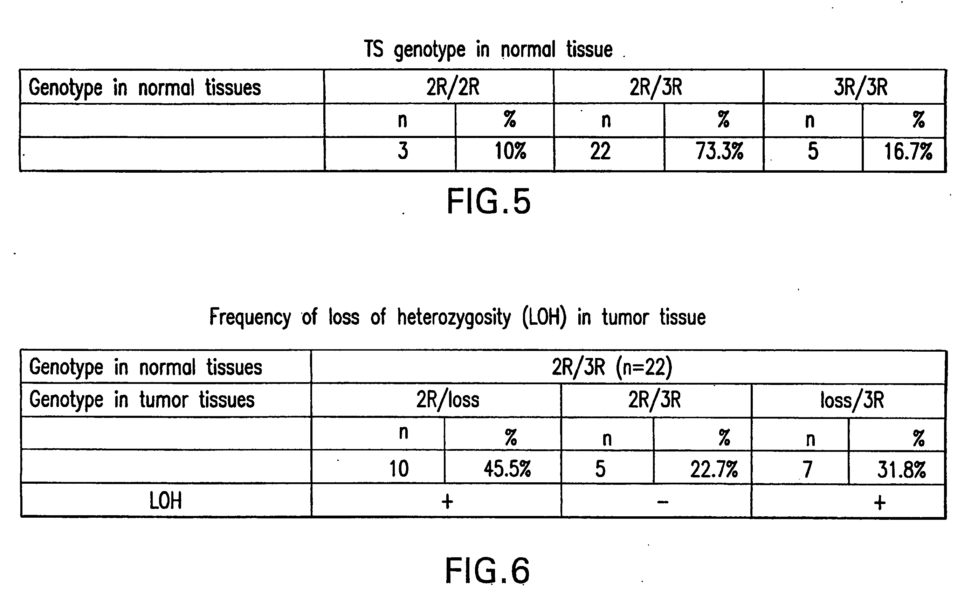

Figure 5 is a table (Table 2) showing the TS genotypes in normal tissue of a study of CRC patients.

Figure 6 is a table (Table 3) showing the frequency of loss of heterozygosity (LOH) in tumor tissue in a study of CRC patients.

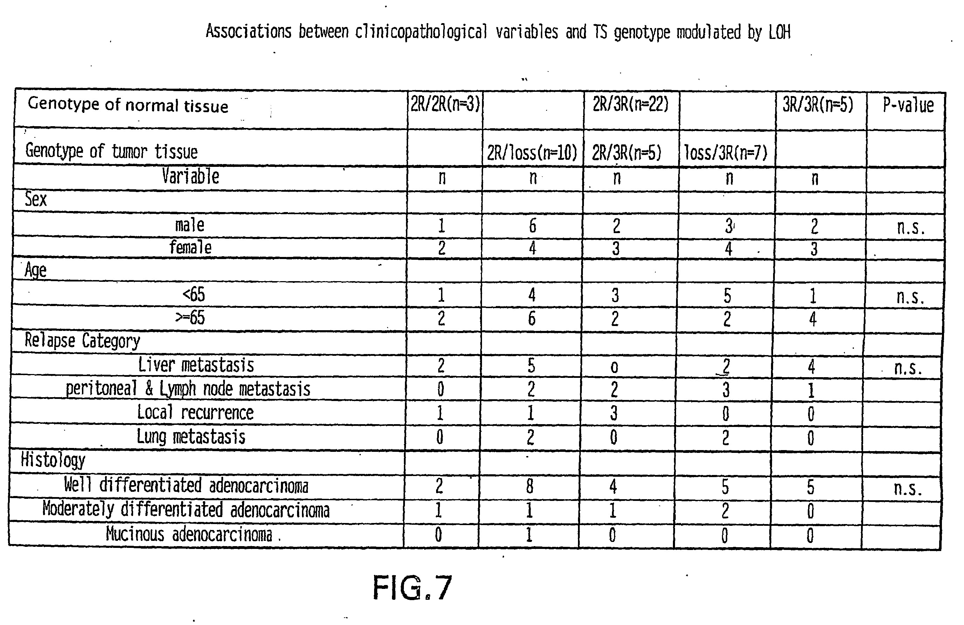

Figure 7 is a table (Table 4) showing the associations between clinicopathological variables and TS genotype modulated by LOH.

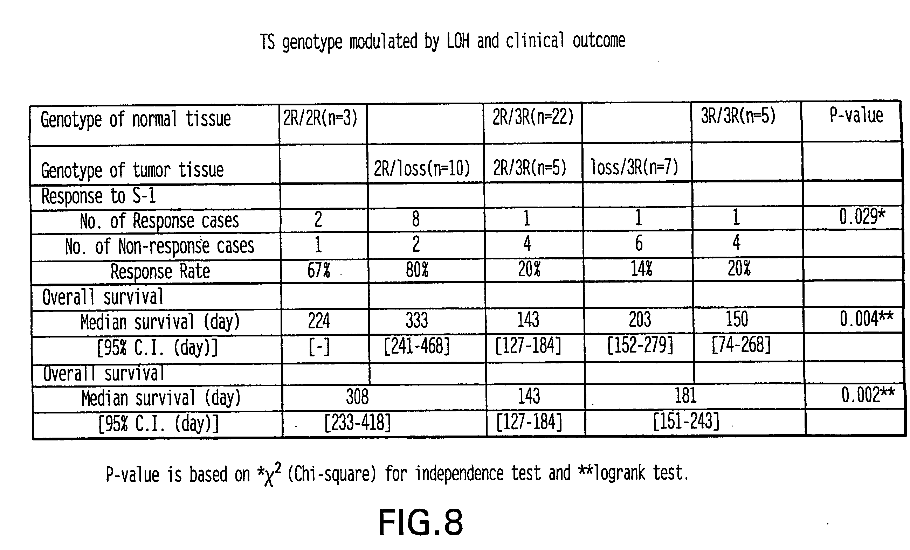

Figure 8 is a table (Table 5) showing the clinical outcome of patients, segregated by TS genotype.

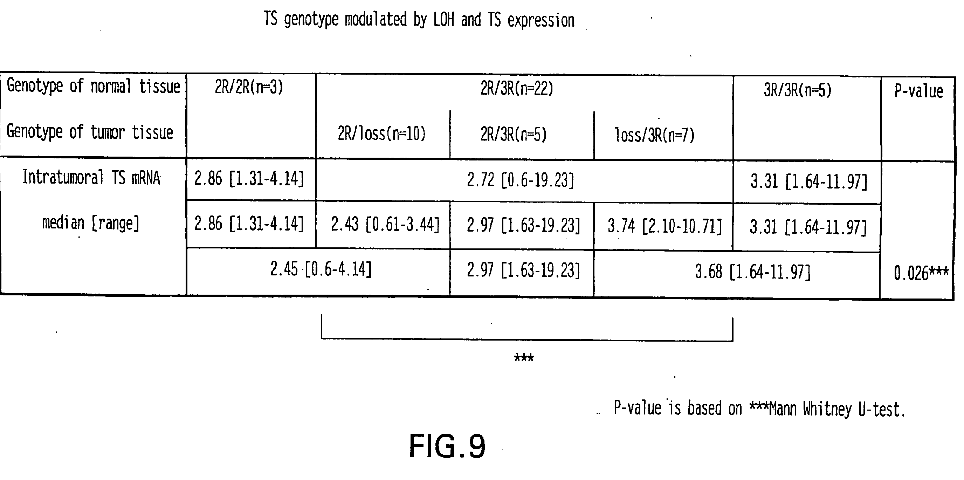

Figure 9 is a table (Table 6) showing TS expression, segregated by TS genotype.

### DETAILED DESCRIPTION OF THE INVENTION

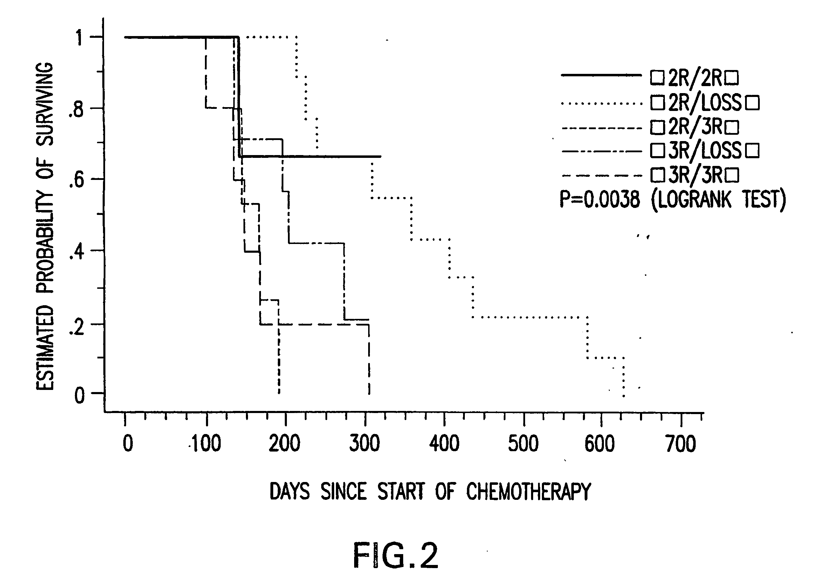

The present invention provides a method of predicting a response to a chemotherapeutic regimen based on loss of heterozygosity at the thymidylate synthase locus in cancer tissue. The TS gene has been localized to the telomeric region of the short arm of chromosome 18, at chromosome band 18p11.32 (Hori, T., et al., Human Genetics, 85(6):576-80, 1990), a region of the genome which is known to undergo deletions in a high percentage of colorectal cancers (Vogelstein B., et al., Science, 244:207-211, 1989). One of the main targets of these deletion events may be the well-known tumor suppressor gene DCC (deleted-in-colorectal cancer) (Klingelhutz AJ, et al., Oncogene 10: 1581-1586, 1995). Due to its proximity to the target gene(s) of chromosome 18 deletions, the TS gene in some cases may be contained within the deleted DNA segment. Indeed, the inventors confirmed in an earlier study that loss of heterozygosity (LOH) occurred frequently at the TS locus in tumor DNA when analysis of the TS genotype from 2R/3R individuals by gel electrophoresis showed a different ratio between 2R and the 3R bands in tumor tissue samples than in normal tissues (i.e., an allelic imbalance) (Kawakami K, et al., Jpn J Cancer Res., 93: 1221-1228, 2002), arising from the fact that upon LOH, a heterozygous 2R/3R genotype would convert to either a 2R/loss or 3R/loss genotype. In this previous study (*Id*) (which is herein incorporated in its entirety be reference), the observed frequency of LOH was 62% (31 of 50) among patients heterozygous for the TS promoter, which is almost identical to that reported by Zinzindohue *et al.* (19/30; 63%) in their group of colorectal cancer (CRC) patients (Zinzindohoue F, et al., J. Clin. Oncol., 19:3442, 2001). The observed LOH frequency in the set of patients of the present study was even higher (77%), which may be ascribed to the use of laser capture microdissection (LCM) of all specimens to isolate the areas of tumor cells as free as possible of any stromal tissue. With this technique for purifying tumor tissue, it is possible to observe quite unambiguously, as demonstrated in Fig. 2, the loss of one or the other TS allele and thus to show definitively the presence or absence of an LOH event at the TS locus.

The present invention is based in part on the observation that the TS locus has a relatively frequent loss of heterozygosity LOH in cancer tissue, which leads to modification of TS genotype in the tumor when it is heterozygous in normal tissue. That is, LOH in individuals who have a heterozygous 2R/3R genotype in their normal tissue would give rise to a tumor with either a 2R/loss or the 3R/loss genotype, depending on which allele became deleted during the LOH event. Thus, if the 3R allele is lost, the tumor has a 2R/loss genotype. If the 2R allele is lost, the tumor has a 3R/loss genotype.

The present invention is based in part on the discovery that tumors acquire the respective chemosensitivities characteristic of 2R/2R and 3R/3R tumors when LOH at the TS locus in tumors of heterozygous 2R/3R and results in either a 2R/loss or a 3R/loss genotype. The salient finding of this study is that the tumor genotype determines the outcome of the therapy: 2R/3R patients with a 2R/loss genotype in the tumor had the same survival as that patients with only 2R genotypes whereas the 2R/loss genotype had strikingly better results from the treatment than patients with the 3R/loss genotype, both in terms of response rate (80% vs. 14%) and survival (333 days vs. 203 days). These results suggest a direct link between TSER polymorphism status in the tumor tissue and response to a TS inhibitor (such as, but not limited to 5-FU, FUdR, UFT, S-1 and capecitabine) based therapy. The data further illustrate that the triple TSER repeat (3R) has a direct negative effect on tumor response as opposed to 2R having a positive effect. As shown in Fig. 2, the survival times of patients who have the tumor 2R/3R genotype are short, similar to those of the 3R-only patients, whereas if the 3R allele is lost during LOH, the resulting 2R/loss tumor has a high response rate and long survival.

The mechanisms by which TSER polymorphic repeats effect tumor response is still unclear. The observation ofHorie et al. (Cell Struct. Funct., 20:191-197,1995) that the expression activity of a reporter gene linked to the TS gene with the double repeat was lower than that of the gene with the triple repeat suggested the possibility that TSER polymorphisms effect tumor sensitivity to 5-FU by regulating TS gene expression. This hypothesis seemed to be confirmed by the results of Pullarkat *et al.* (Pullarkat, S. T., et al., Pharmacogenomics J., 1, 65-70, 2001), who reported a significant TSER-dependent difference (ca. 3.6-fold) in TS gene expressions in colorectal cancer patients and suggested that this difference accounted for the lower response rates of 3R/3R cancer patients compared to 2R/2R genotypes. However, when other data are considered, the association between the number of TSER polymorphisms and TS gene expression does not appear straightforward. For instance, 130 colorectal cancer specimens were analyzed and no significant difference in TS gene expression between 2R/2R and 3R/3R genotypes was found. Instead 3R/3R tumors had a significantly higher TS protein level (Pullarkat, S. T., et al., Pharmacogenomics J., 1, 65-70, 2001), which would also account for lower response rates among 3R/3R patients. This finding suggested that translational activity producing TS protein from mRNA is effected by TSER polymorphism, as is indeed was supported by *in vitro* experiments showing that the 3R promoter caused an increase in translation activity (Id). Etienne *et al.* analyzed 103 CRC tumors treated with 5-FU/folinic acid and found, interestingly, that 2R/3R tumors had higher TS enzyme activity than either 2R/2R or 3R/3R tumors, as well as the shortest survival of the patients.

In the study leading to the present invention, tumors with only 3R genotypes (3R/3R + 3R/loss) showed higher mean *TS* gene expression than those with only 2R genotypes (∼1.5-fold). The finding that 3R/loss genotypes had higher expression than the 2R/loss genotypes (Table 6) by a similar amount suggests that the 3R does exert a direct, albeit modest, up-regulation ofTS gene expression. However, the small difference in mean TS gene expression between tumors bearing only 2R or 3R genotypes seems insufficient to account for the rather striking difference in response to S-1 treatment, suggesting that mechanisms other than (or in addition to) TS regulation may be involved.

For example, several recent studies suggest that *TSER* repeats have a role in regulating the levels of folate metabolites, which could affect drug toxicity to patients and anti-tumor activity of drugs that interact with folate-utilizing enzymes. Significantly lower levels of plasma folates and homocysteine were found in 3R/3R genotype individuals (Trinh, B.N., et al., Hum. Genet., 111:299-302, 2002). This observation might account for the lower toxicity to 5-FU treatment experienced by 3R/3R patients (Pullarkat, S. T., et al., Pharmacogenomics J., 1, 65-70, 2001) because elevated levels of plasma homocysteine have been found to be associated with higher risk of drug toxicity (Niyikiza, C., et al., Molecular Cancer Therapeutics, 1:545-52, 2002). *TSER* repeat status has been linked to response of adult acute lymphoblastic leukemia (ALL) to methotrexate, a dihydrofolate reductase inhibitor, which may also be influenced by folate levels (Krajinovic, M., et al., Lancet, 359: 1033-1034, 2002.). Associations have been noted among *TSER* polymorphisms, folate intake and risk of colorectal cancer (Ulrich, C.M., et al., Cancer Res., 62: 3361-3364, 2002) as well as with overall risk of developing risk of adult ALL. (Skibola, C.F , et al., Blood, 99:3786-91, 2002).

Thus, the present invention is based in part on the discovery that a high percentage of cancer patients undergo LOH at the TS locus in the tumor and those who have a 2R/3R normal tissue genotype segregate into two groups with different tumor TS genotypes, one of whom (the 2R/loss patient) can anticipate considerably better clinical outcome from fluoropyrimidine treatment than the other (the 3R/loss patient). Whether a particular individual will acquire a 2R/loss or a 3R/loss tumor is a matter of chance with an equal probability of ending up with either one. Thus, analysis of TS genotype in tumor tissue, in conjunction with TS gene expression measurements, can help to identify patients who should be considered as good candidates for chemotherapy with TS-directed regimens and those who, due to their lower probability of response, should be considered for another type of treatment.

Thus, the present invention relates to the use of these TS gene polymorphisms as tools for prediction of response and selection of therapy. It is therefore evident that analysis only of normal tissue is insufficient but tumor tissue must also be analyzed to establish the TS polymorphism status in the tumor. In addition, the present invention also contemplates anaylzing normal tissue genotype as it also provides insight into predicting a response to a chemotherapeutic regimen, particularly in predicting drug toxicity levels.

Thus, in predicting a response to a chemotherapeutic regimen the genotype of the tumor tissue is relevant in predicting whether the tumor is likely to respond to a chemotherapeutic regimen. Further the genotype of the non-tumor tissue may be relevant in predicting the risk of developing drug toxicity. Thus, the knowledge of both tissue genotypes assists the physician in developing a suitable chemotherapeutic regimen that will have a greater chance of success in battling the tumor, while also decreasing the risk of drug toxicity.

Accordingly, one aspect of the present invention provides a method of predicting a response to a chemotherapeutic regimen based on loss of heterozygosity at the thymidylate synthase locus in cancer tissue. This method comprises determining the genotype of the tandem repeat polymorphism in the TS gene enhancer region (TSER) of a patient in normal tissue and in the cancerous tissue. The two genotypes are compared to determine whether a loss of heterozygosity at the thymidylate synthase locus has occurred in the tumor tissue. Based on the loss of heterozygosity in the tumor sample, a response to a chemotherapeutic regimen can be predicted. As shown above, a patient having a 2R/loss genotype in the tumor sample will likely have a similar response to the chemotherapeutic regimen as patients having a 2R/2R tumor sample genotype. For example, if patients having a 2R/2R tumor sample respond positively to a chemotherapeutic regimen, then it can be predicted that patients having a 2R/loss tumor genotype will also respond positively to the same chemotherapeutic regimen. Similarly, if patients having a 3R/3R tumor sample respond negatively or show no response to a chemotherapeutic regimen, then it can be predicted that patients having a 3R/loss tumor genotype will also respond negatively or show no response to the same chemotherapeutic regimen.

Further, the genotype of the normal tissue may be used to predict the patient's risk in developing drug toxicity. For example, as stated above studies have shown that 3R/3R patients were found to experience lower risks of drug toxicity than 2R/2R patients. Thus, a 2R/3R normal tissue patient will likely exhibit drug toxicity at doses somewhat lower than 3R/3R patients, but will likely be able to withstand higher doses than 2R/2R patients.

What is meant by the term "response" relates the effects of a chemotherapeutic regimen on tumor tissue or cells. Methods of measuring such response are defined with varying criteria as appropriate, usually depending upon the chemotherapeutic regimen and the type of cancer being treated. Generally, criteria include tumor size reduction, distant site metastases (such as lymph node and lung metastases in the case of colorectal cancer) and others depending upon the cancer tissue type, stage of cancer and metastases and chemotherapeutic regimen. One skilled in the art would appreciate and understand the appropriate criteria for the conditions surrounding the tumor type and the

chemotherapeutic regimen. For example, in one of the studies leading to the present invention, to be classified as a "responder" to a chemotherapeutic regimen, a tumor had to have a 50% reduction in the sum of the products of the perpendicular diameters of the indicator lesion without growth of other disease or the appearance of new lesions. Thus, a positive response in this case would mean tumor reduction and no formation of new lesions. Conversely, a negative response would mean no tumor reduction or even continued tumor growth and/or the appearance of new lesions.

In one embodiment of the present invention the genotype of a patient's normal tissue is also used to predict potential drug toxicity. For example, as stated above studies have shown that 3R/3R patients were found to experience lower risks of drug toxicity to 5-FU than 2R/2R patients. Thus, a 2R/3R normal tissue patient will likely exhibit drug toxicity at doses somewhat lower than 3R/3R patients, but will likely be able to withstand higher doses than 2R/2R patients. Thus, using one of the methods of the present invention to predict a response to a chemotherapeutic regimen, one would predict a patient having a 2R/3R normal tissue genotype and a 2R/loss tumor tissue genotype to react positively to 5-FU treatment (i.e. decrease in tumor size), but be more sensitive to drug toxicity than a patient having a 3R/3R normal tissue genotype. Thus using this prediction, along with compiled data on other similar individuals, a physician could tailor the chemotherapeutic regimen and dosing based on prior patient data relating to tumor responses and drug toxicity.

In determining the genotype of the tumor sample, either fresh or preserved tumor tissue/cells may be used. If preserved, they are preferably preserved as formalin-fixed paraffin embedded (FPE) samples. The FPE tumor sample is then preferably subjected to laser capture micro-dissection (LCM). LCM is useful in isolating tumor cells from non-tumor cells and thus provides a more accurate assessment of tumor tissue genotype.

Kohya et al, International Journal of Molecular Medicine, vol. 11, 2003, pp 65-69 refers to tumor tissue specimens which were fixed with formalin and embedded in paraffin for use in manual-micro-dissection. This article also describes the laser capture micro-dissection of cryopreserved tumor tissue specimens.

TS gene expression levels in the tumor tissue may also be measured. Such measurement may assist in predicting a response to a chemotherapeutic regimen as *in vitro* studies have shown that cells become resistant to TS inhibitors by up-regulating TS expression and raising intracellular TS levels. Studies have shown that TS expression varies considerably among tumors and that sensitivity of various tumors to 5-FU-based chemotherapy is correlated with the intratumoral level of TS. Thus, in addition to determining the tumor genotype, measuring TS gene expression in the tumor may provide useful information in predicting a response to a chemothereapeutic outcome.

To assist in predicting a response, the present invention also contemplates, compiling data regarding patients' tumor and normal tissue genotype and their responses to chemotherapeutic regimens and/or TS gene expression levels in tumor cells. For example, patients whose tumors responded to a chemotherapeutic regimen may be classified as "responders." The genotype of their tumor, as well as TS gene expression levels in their tumors is determined and recorded. Likewise, patients whose tumors did not respond favorably to a chemotherapeutic regimine may be classified as "non-responders." The genotype of their tumor, as well as TS gene expression levels in their tumors is determined and recorded. Then, when a patient is diagnosed with a cancer, the genotype of the tumor as TS gene expression levels in the tumor is tested and compared to the compiled data. If the patient's tumor genotype and TS gene expression levels correlate with the responder group, a positive response may be predicted for that patient. Conversely, when a patient's tumor genotype and TS gene expression levels correlate to the non-responder group, a negative response may be predicted for the chemotherapeutic regimen for that patient.

For example, if a patient "A" has the normal tissue genotype of 2R/3R and the tumor tissue genotype is 2R/loss, using a method of the present invention, one would predict the response to the chemotherapeutic regimen in patient "A" to be similar to a response to the same chemotherapeutic regimen in a patient having a 2R/2R tumor or a 2R/2R normal tissue genotype. As another example, if a patient "B" has the normal tissue genotype of 2R/3R and the tumor tissue genotype is 3R/loss, using a method of the present invention one would predict the response to the chemotherapeutic regimen in patient "B" to be similar to a response to a same chemotherapeutic regimen in patients having a 3R/3R cancer or normal tissue genotype.

Similarly, in predicting drug toxicity, the present invention contemplates compiling data regarding drug toxicity levels in patients, along with the patients' normal tissue and tumor tissue genotype. Thus, a "safe" level of drug administered (before causing lethal drug toxicity) may be predicted based on the patient's normal tissue genotype in comparison with other patients having the same normal tissue genotype and their safe drug dosage levels.

The chemotherapeutic regimen may be any regimen useful in treating the cancer/tumor at issue. One skilled in the art will be knowledgeable in the appropriate chemotherapeutic regimen to choose. Various factors include, but are not limited to, the type of cancer, the patient's status/health, the extent of the cancer, tumor size, etc. In addition, other therapies may also be used simultaneously such as radiation therapy or a chemotherapeutic drug combination. Also the chemotherapeutic regimen may combine other therapies including the use of gene therapies using antisense oligonucleotides, cancer antigen-directed antibodies, co-factors, radiation treatment and the like.

In the case of human gastrointestinal cancers, such as colorectal cancer, TS inhibitors including fluoropyrimidines and folate-based inhibitors, seem to be the current *in vogue* chemotherapies. Thus, the present invention contemplates predicting a response to, but not limited to, TS inhibitors. TS inhibitors include, but are not limited to 5-FU, FUdR, UFT, S-1, capecitabine, ratitrexed, pemetrexed, and nolatrexed, or a combination thereof. Oflen in treating CRC, multiple therapies are involved. These include the administration of additional chemotherapeutic substances, such as cisplatin, oxaliplatin, taxanes as well as radiation treatment.

### EXAMPLES

### Example 1:

### Patient Population

Eligible patients had (a) a diagnosis of disseminated or recurrent colorectal cancer after surgical operation; (b) a Eastern Cooperative Oncology Group performance status of 0 to 2 with adequate hematological, hepatic, and renal function; (c) no treatment during the preceding four weeks; and (d) a lesion that was measurable by radiological examination.

### Treatment

Patients were treated with S-1 twice daily for 28 days, followed by a 2-week period of rest. The S-1 was given orally after breakfast and dinner. As in previous phase II studies (Villafranca, E., et al., J. Clin. Oncol., 19: 1779-1786, 2001; Zinzindohoue, F., et al., J. Clin. Oncol., 19:3442, 2001.), Body surface area (BSA) was used to determine the dose of S-1 administered, as follows: BSA<1.25m², 40mg; 1.25-1.5m², 50mg; >=1.5m², 60mg. These treatments were repeated until disease progression as determined by the treating physician, or at the physician's discretion.

The protocol was reviewed and approved by an institutional review board and an ethics committee before study activation, and informed consent was obtained from every patient according to the institutional regulations.

### Evaluation

After two cycles of treatment, measurable disease was reassessed. Response criteria were the standard definitions used for national cooperative group trials (Green, S. et al., Investig. New. Drugs, 10: 239-253, 1992). Response was assessed by CT in liver, lymph node, and lung metastases, as well as in primary lesions. To be classified as a responder, a tumor had to have a 50% reduction in the sum of the products of the perpendicular diameters of the of the indicator lesion without growth of other disease or the appearance of new lesions (*Id*).

### Microdissection

A representative formalin-fixed, paraffin-embedded pre-S 1 treatment tumor specimen was selected by a pathologist after examination of the hematoxylin and eosin stained slides. Ten micron thick sections were stained with neutral fast red to enable visualization of histology for laser capture microdissection (P.A.L.M. Microlaser Technologies AG, Munich Germany), which was performed to ensure that only tumor cells were studied. Tissue collected from the specimen that had no cancer invasion by histopathology was considered to be normal tissue.

### RNA Isolation and cDNA Synthesis

RNA isolation from paraffin embedded specimens was done according to the procedure set out in U.S. Patent number 6,248,535, which is hereby incorporated by reference in its entirety. Following RNA isolation, cDNA was prepared from each sample as described previously (Lord RV, et al., J. Gastrointest Surg., 4: 135-142, 2000).

### RT-PCR

Relative cDNA quantitation for TS and an internal reference gene (β-actin) was done using a fluorescence based real-time detection method (ABI PRISM 7900 Sequence Detection System [TaqMan®], Applied Biosystems, Foster City, CA), as described previously (Lord RV, et al., J. Gastrointest Surg., 4: 135-142, 2000; Heid CA, et al., Genome Res., 6: 986-994, 1996; Gibson UE, et al., Genome Res., 6: 995-1001, 1996). The primers and probe sequences used are given in Table 1. The PCR reaction mixture consisted of 600 nM of each primer, 200 nM probe, 2.5 U AmpliTaq Gold Polymerase, 200 µM each dATP, dCTP, dGTP, 400 µM dUTP, 5.5 mM MgCl₂, and 1 x Taqman Buffer A containing a reference dye, to a final volume of 25 µl (all reagents Applied Biosystems, Foster City, CA). Cycling conditions were 50°C for 10s, 95°C for 10 min, followed by 46 cycles at 95°C for 15s and 60°C for 1 min. Colon, liver, and lung RNAs (all Stratagene, La Jolla, CA) were used as control calibrators on each plate.

### DNA Extraction, PCR and Electrophoresis

Collected DNA was extracted using the QIAamp Kit (Qiagen, Valencia, CA, USA). The promoter region of the TS gene was amplified by polymerase chain reaction (PCR) using the following primers: forward primer 5'-GCGGAAGGGGTCCTGCCA-3' (SEQ ID NO: 1) and reverse primer 5'-TCCGAGCCGGCCACAGGCAT -3' (SEQ ID NO:2). PCR was performed using the conditions described previously (Kawakami, K., et al., Anticancer Res., 19: 3249-3252, 1999). The PCR products was analyzed by electrophoresis on a 10% TBE-Urea Polyacrylamide gel (Invitrogen Corp., Carlsbad, CA).

### Statistical Analysis

The gene expression values are expressed as ratios between two absolute measurements (gene of interest/internal reference gene). The gene expression values in each TS genotype group were analyzed using the Mann-Whitney U test. Chi-square for independence test was used to assess the association between TS genotype and response to chemotherapy. The log-rank test was used to measure the association between TS genotype and survival.

### Results

A total of 30 primary pre-S 1 treatment colorectal cancer specimens from 30 patients, all of whom had Stage IV disease, were studied. Thirteen of the patients (43.3%) were classified as responders to S-1 and 17 patients (56.7%) were non-responders. TS genotype and TS expressions values were obtained for all of 30 patients. Fourteen of the 30 patients (46.6%) included in the study were male and the median age was 65.0 (range 39-79). The median overall survival time for all 30 patients was 215.5 days (range 98-627). The median overall survival for patients with S-1 responding tumors was 303 days (range 139-627) and for non-responders was 190 days (range 98-435).

### TS polymorphism and LOH in the number of TS repeat sequence in colorectal normal tissue and cancer tissue

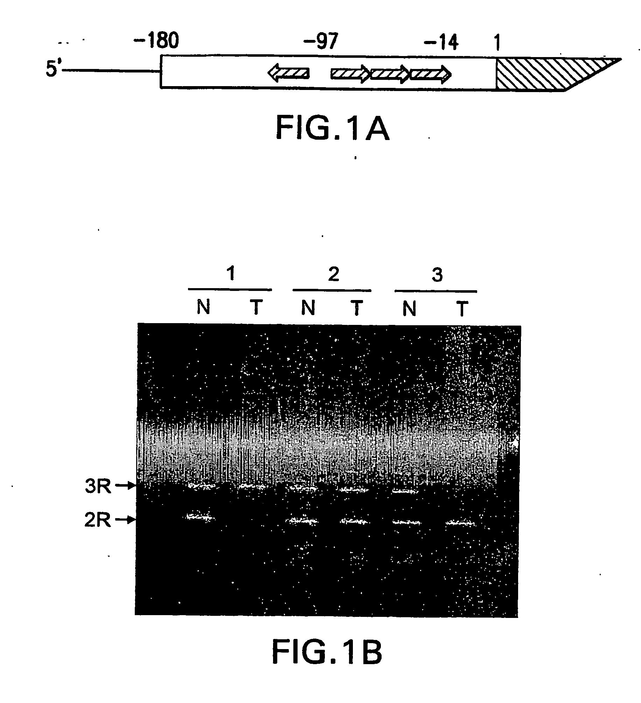

PCR fragments with estimated length of 107 and 135 bp (Fig. 1) were obtained. The 107- and 135-bp fragments represent the two- and three-repeat (2R and 3R) sequence. The TS genotypes were classified into 2R-homozygote (2R/2R), 3R-homozygote (3R/3R), and 2R/3R-heterozygote. The frequency of each genotype in the 30 colorectal normal tissues is shown in Table 2. In the 22 normal tissues with the 2R/3R genotype, the 10 cancer tissues showed only 2R-sequence band (2R/loss), and the 7 cancer tissues showed only 3R-sequence band (3R/loss). See Table 3 and Fig. 1. The each rate of incidence of loss of heterozygosity in the TS locus is 45% (10/22) of 2R/loss genotype and 31.8% (7/22) of loss/3R genotype (Table 3).

### Clinicopathological Characteristics and TS polymorphism modulated by LOH

The 30 patients were divided into five groups depending on TS genotype modulated by LOH. Regarding clinicopathological characteristics, there was no significant difference between these five groups (Table 4). Regarding relapse category, 13 of the all 30 patients (43%) had liver metastasis. The majority of tumors (29/30) were well or moderately differentiated adenocarcinoma histologically.

### TS polymorphism modulated by LOH and response to S-1 chemotherapy

The response rate of the 10 patients with 2R/loss genotype in the cancer is 80% (8/10) and highest in the each genotypes, and the response rate of the 7 patients with 3R/loss genotype is 14% (1/7) and lowest in the each genotypes. There is a significant difference between each response rate (p=0.029) (Table 5).

### TS polymorphism modulated by LOH and overall survival

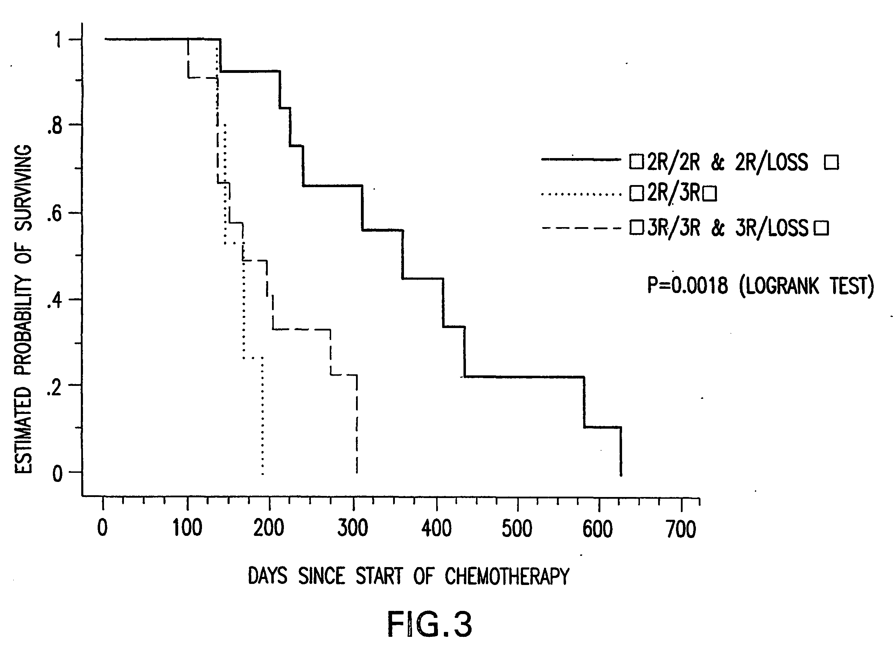

The median overall survival periods were 333days (95% C.I; 241-468 d.) for those colorectal patients with 2R/3R genotype in their normal tissue and 2R/loss genotype in cancer tissue. This survival is significantly longer than that of patients with other genotypes (p=0.004) (Table 4 and Fig. 2). The median overall survival periods were 308 days (95% CJ; 233418 d.) for colorectal patients with 2R/2R or 2R/loss genotype in the cancer tissue. This is significantly longer than that of patients with other genotypes in the cancer tissue (p=0.002) (Table 5 and Fig. 3).

### TS polymorphism modulated by LOH and intratumoral TS mRNA expression

The median intratumoral TS mRNA expression were 2.45 (range 0.6-4.14) for patients with 2R/2R or 2R/loss genotype in the cancer tissue, 2.97 (range 1.63-19.23) for patients with 2R/3R genotype in the cancer tissue, and 3.68 (range 1.64-11.97) for patients with loss/3R or 3R/3R genotype. The TS genotype modulated by LOH in the cancer tissue was statistically associated with intratumoral TS mRNA expression (p=0.026) (Table 6).

## Claims

1. A method of predicting a cancer patient's response to a chemotherapeutic regimen, wherein the regimen comprises a thymidylate synthase (TS) inhibitor, said method comprising:
(a) determining the genotype of the tandem repeat polymorphism in the TS gene enhancer region (TSER) in the patient's normal tissue;
(b) determining said genotype in the patient's tumor tissue;
(c) comparing the normal tissue genotype with the tumor tissue genotype to determine whether a loss of heterozygosity at the TSER has occurred in the tumor tissue; and
(d) if there is a loss of heterozygosity, predicting a response to the chemotherapeutic regimen based on the loss of heterozygosity in the tumor sample, wherein
(i) a determination of the presence of a triple TSER repeat (3R) allele predicts a direct negative effect on the response of the tumor to the chemotherapeutic regimen;
(ii) a determination of loss of heterozygosity from 2R/3R in normal tissue to 2R/loss in tumor tissue predicts increased sensitivity of the tumor to the chemotherapeutic regimen relative to the sensitivity of a tumor having a 2R/3R genotype; and/or
(iii) a determination of loss of heterozygosity from 2R/3R in normal tissue to 3R/loss in tumor tissue predicts a sensitivity of the tumor to the chemotherapeutic regimen similar to that of a tumor having a 3R/3R genotype.

2. The method of claim 1, wherein the tumor tissue is formalin-fixed paraffin-embedded (FPE) tissue.

3. The method of claim 2, further comprising subjecting the FPE tumor tissue to laser capture micro-dissection.

4. The method of claim 1, further comprising measuring TS gene expression levels in the tumor tissue.

5. The method of claim 4, further comprising
(a) determining levels of TS gene expression in tumor cells of responder patients, said responder patients having tumors that respond to the chemotherapeutic regimen;
(b) determining levels of TS gene expression in tumor cells of non-responder patients, said non-responder patients having tumors that show no response to the chemotherapeutic regimen;
(c) comparing levels of TS gene expression in tumor tissue of said patient with TS gene expression levels of responder and non responder patients; and
(d) predicting a response to the chemotherapeutic regimen based on the comparison of step (c).

6. The method of claim 1, wherein the chemotherapeutic regimen comprises administration of 5-FU, FUdR, UFT, S-1, capecitabine, ratitrexed, pemetrexed, or nolatrexed, or a combination thereof.

7. The method of claim 6, wherein the chemotherapeutic regimen comprises 5-FU, FUdR, UFT, S-1, capecitabine, ratitrexed, pemetrexed, or nolatrexed in combination with cisplatin, oxaliplatin, a taxane, or radiation.

8. The method of claim 1, further comprising
(a) identifying other patients having a normal tissue genotype that is the same as the normal tissue genotype of the cancer patient;
(b) calculating drug toxicity levels in said other patients after treatment with the chemotherapeutic regimen; and
(c) correlating said drug toxicity levels to the cancer patient to predict the patient's response to the chemotherapeutic regimen.

## Patentansprüche

1. Verfahren zum Prognostizieren einer Reaktion eines Krebspatienten auf eine chemotherapeutische Behandlung, wobei die Behandlung einen Thymidylatsynthase-(TS-) Inhibitor umfasst, wobei man bei dem Verfahren:
(a) den Genotyp des Tandemwiederholungs-Polymorphismus in der TS-Genverstärkerregion (TSER) in dem normalen Gewebe des Patienten bestimmt,
(b) den Genotyp in dem Tumorgewebe des Patienten bestimmt,
(c) den Genotyp des normalen Gewebes mit dem Genotyp des Tumorgewebes vergleicht, um festzustellen, ob ein Verlust an Heterozygotie bei der TSER in dem Tumorgewebe aufgetreten ist, und
(d) wenn ein Verlust an Heterozygotie vorliegt, eine Reaktion auf die chemotherapeutische Behandlung vorhersagt auf der Grundlage des Verlusts an Heterozygotie in der Tumorprobe, wobei
(i) eine Feststellung der Gegenwart eines dreifachen TSER-Wiederholungs-(3R-)Allels eine unmittelbar negative Wirkung auf die Reaktion des Tumors auf die chemotherapeutische Behandlung prognostiziert,
(ii) eine Feststellung des Verlusts an Heterozygotie von 2R/3R in normalem Gewebe zu 2R/Verlust in Tumorgewebe eine erhöhte Empfindlichkeit des Tumors auf die chemotherapeutische Behandlung in Bezug auf die Empfindlichkeit eines Tumors mit einem 2R/3R-Genotyp prognostiziert und/oder
(iii) eine Feststellung eines Verlusts an Heterozygotie von 2R/3R in normalem Gewebe zu 3R/Verlust in Tumorgewebe eine Empfindlichkeit des Tumors auf die chemotherapeutische Behandlung prognostiziert, die vergleichbar zu der eines Tumors mit einem 3R/3R-Genotyp ist.

2. Verfahren nach Anspruch 1, wobei das Tumorgewebe ein in Formalin fixiertes und in Paraffin eingebettetes (FPE) Gewebe ist.

3. Verfahren nach Anspruch 2, bei dem man außerdem das FPE-Tumorgewebe einer Lasereinfang-Mikrosektion unterwirft.

4. Verfahren nach Anspruch 1, welches außerdem umfasst, dass man die Expressionsniveaus des TS-Gens in dem Tumorgewebe misst.

5. Verfahren nach Anspruch 4, welches außerdem umfasst:
(a) Bestimmung der TS-Genexpressionsniveaus in Tumorzellen von ansprechenden Patienten, wobei die ansprechenden Patienten Tumore haben, die auf die chemotherapeutische Behandlung ansprechen,
(b) Bestimmung der TS-Genexpressionsniveaus in Tumorzellen von nicht-ansprechenden Patienten, wobei die nicht-ansprechenden Patienten Tumore haben, die keine Reaktion auf die chemotherapeutische Behandlung zeigen,
(c) Vergleich der TS-Genexpressionsniveaus in Tumorgewebe von dem Patienten mit TS-Genexpressionsniveaus von ansprechenden und nicht-ansprechenden Patienten und
(d) Prognostizieren einer Reaktion auf die chemotherapeutische Behandlung auf der Grundlage des Vergleichs von Stufe (c).

6. Verfahren nach Anspruch 1, wobei die chemotherapeutische Behandlung die Verabreichung von 5-FU, FUdR, UFT, S-1, Kapezetabin, Ratitrexed, Pemetrexed oder Nolatrexed oder einer Kombination davon umfasst.

7. Verfahren nach Anspruch 6, wobei die chemotherapeutische Behandlung 5-FU, FUdR, UFT, S-1, Kapezetabin, Ratitrexed, Pemetrexed oder Nolatrexed in Kombination mit Cisplatin, Oxaliplatin, einem Taxan oder Bestrahlung umfasst.

8. Verfahren nach Anspruch 1, welches weiterhin umfasst:
(a) Identifizieren von anderen Patienten mit einem Genotyp von normalem Gewebe, der zu dem Genotyp von normalem Gewebe des Krebspatienten gleich ist,
(b) Berechnen des Arzneistoff-Toxizitätsniveaus in dem anderen Patienten nach der Behanldung mit der chemotherapeutischen Behandlung und
(c) Korrelieren der Arzneistoff-Toxizitätsniveaus mit dem Krebspatienten, um die Reaktion des Patienten auf die chemotherapeutische Behandlung zu prognostizieren.

## Revendications

1. Procédé permettant de prédire la réponse d'un patient cancéreux à un régime chimiothérapeutique, où le régime comprend un inhibiteur de la thymidylate synthase (TS), ledit procédé comprenant :
(a) la détermination du génotype du polymorphisme de séquences en tandem répétées dans la région amplificatrice du gène TS (TSER) dans le tissu normal du patient ;
(b) la détermination dudit génotype dans le tissu tumoral du patient ;
(c) la comparaison du génotype du tissu normal au génotype du tissu tumoral pour déterminer s'il s'est produit une perte d'hétérozygosité au niveau de la TSER dans le tissu tumoral ; et
(d) s'il y a perte d'hétérozygosité, la prédiction d'une réponse au régime chimiothérapeutique basée sur la perte d'hétérozygosité dans l'échantillon tumoral, où
(i) une détermination de la présence d'un allèle à triple répétition dans la TSER (3R) prédit un effet négatif direct sur la réponse de la tumeur au régime chimiothérapeutique ;
(ii) une détermination de perte d'hétérozygosité de 2R/3R dans le tissu normal en 2R/perte dans le tissu tumoral prédit une augmentation de la sensibilité de la tumeur au régime chimiothérapeutique par rapport à la sensibilité d'une tumeur ayant un génotype 2R/3R ; et/ou
(iii) une détermination de perte d'hétérozygosité de 2R/3R dans le tissu normal en 3R/perte dans le tissu tumoral prédit une sensibilité de la tumeur au régime chimiothérapeutique similaire à celle d'une tumeur ayant un génotype 3R/3R.

2. Procédé selon la revendication 1, dans lequel le tissu tumoral est un tissu fixé au formol et noyé dans de la paraffine (FPE).

3. Procédé selon la revendication 2, comprenant en outre la soumission du tissu tumoral FPE à une microdissection par capture laser.

4. Procédé selon la revendication 1, comprenant en outre la mesure des taux d'expression du gène TS dans le tissu tumoral.

5. Procédé selon la revendication 4, comprenant en outre
(a) la détermination des taux d'expression du gène TS dans les cellules tumorales des patients répondeurs, lesdits patients répondeurs ayant des tumeurs qui répondent au régime chimiothérapeutique ;
(b) la détermination des taux d'expression du gène TS dans les cellules tumorales des patients non répondeurs, lesdits patients non répondeurs ayant des tumeurs qui ne montrent aucune réponse au régime chimiothérapeutique ;
(c) la comparaison des taux d'expression du gène TS dans le tissu tumoral dudit patient aux taux d'expression du gène TS des patients répondeurs et non répondeurs ; et
(d) la prédiction d'une réponse au régime chimiothérapeutique basée sur la comparaison de l'étape (c).

6. Procédé selon la revendication 1, dans lequel le régime chimiothérapeutique comprend l'administration de 5-FU, de FUdR, de UFT, de S-1, de capécitabine, de ratitrexed, de permetrexed ou de nolatrexed, ou d'une combinaison de ceux-ci.

7. Procédé selon la revendication 6, dans lequel le régime chimiothérapeutique comprend du 5-FU, du FUdR, du UFT, du S-1, de la capécitabine, du ratitrexed, du permetrexed ou du nolatrexed en combinaison avec du cisplatine, de l'oxaliplatine, un taxane ou des rayons.

8. Procédé selon la revendication 1, comprenant en outre
(a) l'identification d'autres patients ayant un génotype de tissu normal qui est identique au génotype de tissu normal du patient cancéreux ;
(b) le calcul des taux de toxicité des médicaments chez lesdits autres patients après traitement avec le régime chimiothérapeutique ; et
(c) la corrélation desdits taux de toxicité des médicaments envers le patient cancéreux pour prédire la réponse du patient au régime chimiothérapeutique.
